# EUROPEAN PATENT APPLICATION

(11) **EP 1 564 198 A1**
(43) Date of publication of application: **17.08.2005**
(21) Application number: 04100506.7
(22) Date of filing: 11.02.2004
(51) Int. Cl.: C07C 2/32, C08F 10/02, C08F 4/69, C08F 4/00

(54) **Heterogeneous chromium catalysts**

(71) Applicant: TOTAL PETROCHEMICALS RESEARCH FELUY, 7181 Seneffe (Feluy) (BE)
(72) Inventor: Nenu, Cristina, 3434 HS Nieuwegein (NL); Weckhuysen, Bert, 3992 KK Houten (NL); Bodart, Philippe, 4480 Clermont sous Huy (ENGIS) (BE)

(57) **Abstract**

The present invention relates to a heterogeneous chromium catalyst system for the polymerisation of ethylene and/or alpha olefins prepared by the steps of:
a) providing a silica-containing support,
b) treating the silica-containing support with a chromium compou nd to form a chromium-based silica-containing support,
c) activating the chromium -based silica-containing support,
d) chemically reducing the activated chromium -based silica-containing support to produce a precursor catalyst,
e) reacting the reduced precursor catal yst with a tridentate ligand compound to obtain a catalyst and
f) optionally activating the catalyst issued from step e) with a cocatalyst.

## Description

The present invention relates to heterogeneous catalysts for the polymerisation of alpha olefins, particularly for the polymerisation of ethylene to hexene. The present invention further relates to methods for producing the catalysts and to the use of such catalysts

A variety of homogeneous catalysts are known in the literature for the polymerisation of alpha olefins. These systems, as referred here below, are based on chromium complexes comprising a chromium core bound to a ligand that comprises electron donors. Nitrogen is the most common electron donor but phosphorus, oxygen or sulphur have also been used. However, a major drawback of these known homogeneous complexes lies in that at the end of the polymerisation, it is difficult to separate these complexes from the components.

Patent WO03/7175 relates to catalyst systems comprising chromium pyrrolide complexes and metal alkyl compound. Said catalyst systems were reported to be active in ethylene trimerization.

Patent WO03/084902 relates to homogeneous multi-dentate chromium complexes activated with alkylaluminoxane in order to oligomerize olefins. The chromium systems can have different ligands, such as 1,3,5 triazacyclohexane with different substituents, cyclopentadienyl ligands with different substituents and N-methylimidazole ligands reported by Ruther T. et al., Organometallics 2001, volume 20, pages 1247 -1250.

Patent WO03/076368 discloses a method for the oligomerization of alpha olefins comprising at least 3 carbon atoms wherein the olefin is brought into contact with a catalyst sytem obtained from a chromium source, a 1,3,5-trialkyl-1,3,5-triazacyclohexane and from at least one activator comprising a boron compound.

Patent WO03/076367 discloses a method for the oligomerization of olefins in presence of a homogeneous catalyst based on 1,3,5 triazacyclohexane -CrCl₃ complex with 1,3,5 triazacyclohexane substituted in 1,3,5 positions with cycloalkylalkyl groups. An alkyl aluminium compound or an alkylalumoxane is used as activator.

R.D. Köhn et al. disclose in Chemical Communications, 2000, pages 1927 - 1928 substituted 1,3,5-triazacyclohexane chromium (III) homogeneous complexes activated with methylalumoxane or N,N-dimethylanilinium tetrakis(pentafluorophenyl) borate and triisobutylaluminium. These complexes are active in the polymerisation of ethylene. The main product is polyethylene, although 1-hexene and some decenes as co-trimers of 1-hexene and ethene were also found in the reaction mixture.

It is an object of the present invention to provide a method for producing a heterogeneous chromium catalyst system for the polymerisation of ethylene and/or alpha olefins.

In the present invention, polymerisation means a chemical process that combines two or more monomers to form a polymer.

In the present invention, hexene includes 1-hexene.

The present inventors have found a method for producing such heterogeneous chromium catalyst system, said method comprising the steps of:
a) providing a silica-containing support,
b) treating the silica-containing support with a chromium compound to form a chromium-based silica-containing support,
c) activating the chromium-based silica-containing support,
d) chemically reducing the activated chromium-based silica-containing support to produce a precursor catalyst,
e) reacting the reduced precursor catalyst with a tridentate ligand compound to obtain a catalyst and
f) optionally activating the catalyst issued from step e) with a cocatalyst.

The present inventors have found that the method according to the invention solves the object of the invention.

The present invention further provides heterogeneous chromium catalysts.

Finally, the present invention provides a process for the polymerisation of ethylene and/or alpha olefins, preferably for the polymerisation of ethylene to hexene, by passing the ethylene and /or alpha olefins over the heterogeneous chromium catalyst system of the invention and by further recovering the polymer produced. The inventors have surprisingly found that the heterogeneous chromium catalyst system has an improved activity by catalytic site.

The silica-containing support material used in the catalyst of the invention may be composed of 5 to 100 weight percent silica, the remainder if any being selected from aluminium oxide, ma gnesium oxide, titanium oxide, zirconium oxide, or mixtures thereof. Preferably, the support is silica.

The specific surface area of the silica-containing support is preferably larger than 200 m²/g and its pore volume greater than 1 cm³/g.

The particles size of the silica-containing support preferably ranges from 0.020 to 2 millimetres. More preferably, the silica-containing support is sieved to achieve size fractions between 0.2 and 0.4 millimetres. The required size fraction can be obtained by any method known in the art. As an example, to obtain size fractions between 0.2 and 0.4 millimetres, the silica-containing support is first press into a wafer, then grained and sieved using size sieves with 0.425 mm and 0.212 mm aperture. This may be done either before the treatment of the silica-containing support with the chromium compound or after it.

The silica-containing support is then treated with a chromium compound. The chromium compound used in the present invention is preferably chromium trioxide. Preferably, the resultant catalyst precursor contains between 0.01 and 2 weight percent of chromium, preferably between 0.1 and 1 weight percent of chromium, based on the total weight of the chromium and support. Most preferably, the catalyst contains 1 weight percent of chromium, based on the total weight of the chromium and support.

The catalyst precursor is then subjected to an activation step in air, at an elevated temperature, for at least 6 hours. The activation temperature preferably ranges from 400 to 900°C. Most preferably, the activation temperature is about 720°C.

After activation, a chemical reduction process occurs at a temperature of from 200°C to 600°C, preferably at a temperature from 350 to 450°C, and more preferably at a temperature of about 4 00°C, in either carbon monoxide or hydrogen.

After reduction, the catalyst precursor is treated with a tridentate ligand compound that has been dissolved in a solvent such as chlorinated solvents, one can cite dichloromethane. Preferably, the tridentate ligand compound is a cyclic tridentate ligand with electron donating atoms in the cycle, substituted or unsubstituted, more preferably, the tridentate ligand compound is a neutral cyclic tridentate ligand with electron donating atoms in the cycle, substitu ted or unsubstituted, most preferably the tridentate ligand compound is the 1,3,5-triazacyclohexane, wherein each of the nitrogen atoms of the 1,3,5-triazacyclohexane may independently be substituted or not. When substituted, the substituents may be hydrocarbon radicals. Preferably the substituents are benzyl. In the present invention, the 1,3,5-tribenzyl 1,3,5-triazacyclohexane is preferably used.

After treatment with the tridentate ligand compound, the catalyst is optionally activated. Depending on the tridentate ligand compound used, the man in the art has to test if the catalyst has to be activated, or not, with a cocatalyst. When the catalyst has to be activated, the activation compound is preferably a fluorinated boron compound and/or an aluminium co mpound.

When 1,3,5-triazacyclohexane substituted or unsubstituted is used as tridentate ligand compound, the catalyst is activated with a cocatalyst. Preferably the catalyst is first activated with a fluorinated boron compound and then with an aluminium compound. In the present invention, the aluminium compound is preferably triisobutylaluminium and the fluorinated boron compound is preferably N,N -dimethylanilinium tetrakis(pentafluorophenyl)borate.

### Examples:

### Example 1: preparation of the heterogeneous catalyst according to the invention.

10 g silica support was introduced in a 50 ml flask. A solution of chromium trioxide prepared by dissolving 0.2 g of chromium trioxide in 20 ml water was added to the support at room temperature in order to get a chromium content of 1 weight percent of chromium based on the total weight of the chromium and the support. After impregnation, the chromium silica-containing support is dried in air at around 150 °C, then sieved in order to achieve fractions from 0.2 to 0.4 mm and loaded in a sealed vessel where the activation treatment took place under dry air for 6 hours at 720°C. After the activation step, the chromium silica-containing support was further reduced under carbon monoxide for thirty minutes at 400°C. In a glove box under inert atmosphere, 1.2 g of the reduced precursor catalyst was placed in a 5 ml flask to which were added 2 ml solution of 0.04 M 1,3,5-tribenzyl 1,3,5-triazacyclohexane ligand prepared by dissolving 1.43 g of 1,3,5-tribenzyl 1,3,5-triazacyclohexane ligand in 100 ml of extra dry dichloromethane. The reduced precursor catalyst and the ligand reacted for 10 minutes at room temperature. In order to remove the un-reacted ligand, the catalyst was washed at room temperature with 10 to 15 ml of dichlorom ethane. The dichloromethane was further removed by evaporation at 40°C. A light purple catalyst powder was produced. The catalyst was further activated with 1.3 to 2 equivalents of N,N -dimethylanilinium tetrakis(pentafluorophenyl) borate prepared by adding 5 mg of said fluorinated boron compound to 2.5 ml of dichloromethane. The solvent was removed from the catalyst by evaporation at 40°C. Afterwards, the catalyst was still activated with 25 to 50 equivalents of triisobutylaluminium.

Before activation with the N,N-dimethylanilinium tetrakis(pentafluorophenyl) borate, the heterogeneous catalyst produced according to the invention and identified in the description as catalyst (Bz)₃TAC-Cr/SiO₂ (with Bz = benzyl) was characterised by using ultraviolet -visible spectroscopy. The UV-Vis absorption bands of the catalyst according to the invention, as well as of the homogeneous complex 1,3,5-tribenzyl 1,3,5-triazacyclohexane chromium trichloride reported in the literature by Köhn et al., identified as homogeneous (Bz)₃TAC-CrCl₃ in the description, and of the reduced precursor catalyst not yet treated with the 1,3,5-tribenzyl 1,3,5-triazacyclohexane ligand were also determined. The values are displayed in Table I.

**Table I**

| **Sample** | **Cr**^{**2+**} **(cm**^{**-1**}**)** | **Cr**^{**3+**} **(cm**^{**-1**}**)** | | |
|---|---|---|---|---|
| Catalyst (Bz)₃TAC-Cr/SiO₂ | - | 13500 | 20700 | 30500 |
| Homogeneous (Bz)₃TAC-CrCl₃ | - | 13900 | 19600 | 29900 |
| Precursor catalyst not treated with the ligand | 10300 | 14000 | 20000 | 28700 |

The UV-Vis spectrum of the catalyst according to the invention showed three major maxima located respectively at 13500 cm⁻¹, 20700 cm⁻¹ and 30500 cm⁻¹. These positions are characteristic for the chromium trivalent d-d transitions that occur in the complex. These positions are not present in the spectrum of the reduced precursor catalyst that has not been treated with the ligand. In the catalyst (Bz)₃TAC-Cr/SiO₂, the maxima are shifted comparing with the homogeneous (Bz)₃TAC-CrCl₃ complex because the silica lattice replaced the chlorine ligands from the homogeneous complex. The UV-Vis spectrum of the homogeneous (Bz)₃TAC-CrCl₃ complex presented also the characteristic 3 d-d transitions of Cr³⁺ in Cr complexes with nitrogen ligands.

Some reagents such as water or mineral acids, in particular hydrochloric acid (HCl), can affect the stability of the catalyst according to the invention. This was tested by treating the catalyst with hydrochloric acid or water by injecting 2 ml of hydrochloric acid or water inside the spectroscopic cell. After 10 min contacting time, the UV-Vis spectra were determined. The UV-Vis absorption bands of the catalyst of the invention, of the catalyst of the invention after treatment with HCl and H₂O as well as of the homogeneous catalyst values are displayed in Table II.

**Table II**

| **Sample** | **Cr**^{**3+**} **(cm**^{**-1**}**)** | | |
|---|---|---|---|
| Catalyst (Bz)₃TAC-Cr/SiO₂ | 13500 | 20700 | 30500 |
| Catalyst (Bz)₃TAC-Cr/SiO₂ treated with HCl | 14520 | 20100 | 28000 |
| Catalyst (Bz)₃TAC-Cr/SiO₂ treated with H₂O | 16400 | 24500 | 27800 |
| Homogeneous (Bz)₃TAC-CrCl₃ | 13900 | 19600 | 29900 |

The new maxima positions of the catalyst of the invention treated with HCl were closer to the positions of the homogeneous complex (Bz) ₃TAC-CrCl₃. Indeed, after the treatment, the Cr-O bond of the catalyst of the invention was broken and the supported complex was converted into the homogeneous complex. The colour of the treated catalyst intensified from light purple to dark purple, which is the colour of the homogeneous complex. The spectrum of the catalyst of the invention treated with water differed also widely from those of the untreated catalyst indicating the presence of new species.

To elucidate the structure of the catalyst of the invention in more detail, fluorescence Extended X-ray Absorption Fine Structure Spectroscopy (EXAFS) data were recorded. The EXAFS measurements involved synchrotron radiation provided by HASYLAB, Hamburg, Germany. The results were compared with the results for a comparable catalyst provided by X-ray diffraction (XRD), that were furnished by CSD database, Cambridge University.

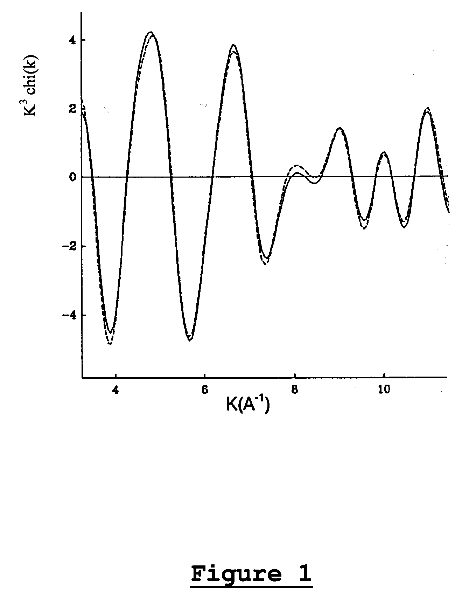

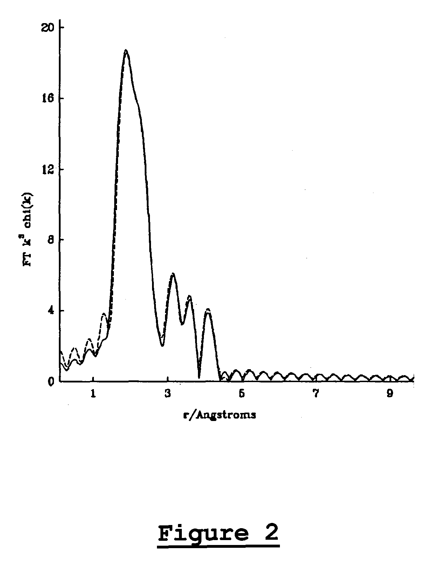

The EXAFS pattern of the heterogeneous chromium catalyst, show n in figure 1 indicated a very good fit of the theoretical curve (dashed line) with this related to the catalyst of the invention (unbroken line). The Fourier transform plot illustrated in figure 2, wherein the dashed and unbroken lines have the same meaning as in figure 1, gave indications about the atom position distribution. Short-range distances were selected in order to visualize the closest atoms around the Cr centre. The distance evaluation is very accurate and is in agreement with data reported by X RD. Table III presents the molecular structure of the catalyst of the invention obtained by EXAFS and the molecular structure reported for the homogeneous catalyst (Bz) ₃TAC-CrCl₃.

**Table III**

| **Coordination Shell** | **Atom** | **Coordination number** | **R**_{**EXAFS**}**(Å) for catalyst of the invention** | **R**_{**XRD**}**(Å) for the homogeneous catalyst** |
|---|---|---|---|---|
| 1^{st} shell | O | 0.9 | 1.908 | - |
| 2^{nd} shell | N | 2.8 | 2.091 | 2.095 |
| 3^{rd} shell | C | 3.4 | 2.601 | 2.615 |
| 4^{th} shell | Si | 1.0 | 3.217 | - |
| Å: Angstroms | | | | |

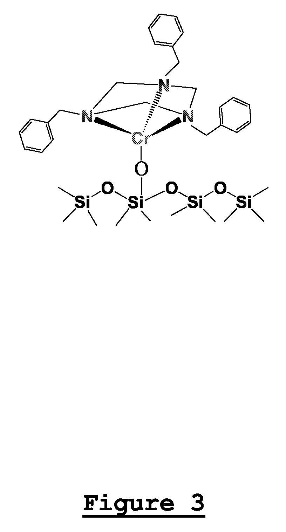

The closest atom to the Cr centre is one oxygen atom, at a very short distance, which implies a very strong bond. Consequently, the anchored complex is linked via one oxygen atom with the silica surface. Three nitrogen atoms are found also around Cr centre at the same distance reported for the reference catalyst as well as for the carbon atoms. Further, a silicon atom is present, in fact the linkage of oxygen with the surface. According to these data, we can conclude that an anchored complex was formed on the silica surface and the molecular structure of the heterogeneous chromium catalyst of the invention is illustrated in figure 3.

In order to confirm that the heterogeneous complex was well anchored onto the surface of the silica with the method described by this invention, a polymerisation was conducted by using two reactors. The catalyst prepared as described hereabove was introduced in a first reactor. Toluene was used as diluent in the reactor and ethylene was continuously added in the reactor at a rate of 30 ml/min. The polymerisation was performed under slurry conditions at normal pressure and at room temperature for 30 minutes. After 30 minutes, the solid phase was separated from the liquid phase. The separation was fulfilled with a double pointed needle and the liquid phase was transferred from the first reactor to a second one. In the second reactor, ethylene was added at a rate of 30 ml/min. No more catalyst was added in the second reactor. After 30 minutes, no catalytic activity occurred in the second reactor indicating the absence of any active homogeneous complex in th e solution, which was transferred from the first reactor to the second reactor. This was confirmed by the atomic absorption spectroscopy of the liquid solution. Indeed, no significant traces of chromium were detected in the liquid solution.

### Example 2: Catalysis of the heterogeneous catalyst according to the invention.

### Example 2.1

The catalyst of the invention prepared according to example 1 was carried out under slurry conditions at normal pressure and at a temperature of 90°C during 60 min. The polymerisation was performed in a slurry reactor connected to a bubbler. The catalyst of the invention was introduced into the reactor. Toluene was used as diluent in the reactor and in the bubbler. Ethylene was continuously added in the reactor at a rate of 30 ml /min. Hexene was determined in the liquid phase of the reactor and of the bubbler as well as in the gas phase coming out from the bubbler by gas chromatography and gas chromatograph-mass spectrometry. The distribution of the hexene (g) in the reactor, bubbler and gas phase are shown in Table IV.

### Example 2.2

In this example, the polymerisation of ethylene to hexene was conducted in the same conditions as in example 2.1, except that the temperature was at room temperature. The distribution of the hexene (g) in the reactor, bubbler and gas phase are shown in Table IV.

### Example 2.3 (comparative)

In this example, the polymerisation of ethylene to hexene was conducted in the same conditions as in example 2.1 excepted that the polymerisation occurred at the room temperature in the presence of the homogeneous complex (Bz)₃TAC-CrCl₃ used for comparison. The distribution of the hexene (g) in the reactor, bubbler and gas phase are shown in Table IV. The activity of the catalyst of the invention was determined through the turnover number (TON), defined as the number of moles of hexene divided by the number of moles of chromium. TON represents the number of molecules of hexene formed on a chromium site. The amount of hexene formed, and the TON for the different Cr complexes are given in Table IV.

**Table IV**

| **Example** | **2.1 (Invention)** | **2.2 (Invention)** | **2.3 (Comparative)** |
|---|---|---|---|
| Reactor | 0.18 | 0.19 | 0.25 |
| Bubber | 0.010 | 0.035 | 0.014 |
| Gas phase | 0.00021 | 0.00013 | 0.00017 |
| hexene (g) | 0.19 | 0.22 | 0.24 |
| TON | 7.9 | 9.6 | 2.9 |

Table IV showed that the heterogeneous catalyst of the invention has a higher polymerisation activity per catalyst site than those of the homogeneous catalyst according to Köln et al. used herein as comparative. This result was unexpected.

## Claims

1. A method for producing a heterogeneous chromium catalyst system, said method comprising the steps of:
a) providing a silica-containing support,
b) treating the silica-containing support with a chromium compound to form a chromium-based silica-containing support,
c) activating the chromium-based silica-containing support,
d) chemically reducing the activated chromium -based silica-containing support to produce a precursor catalyst,
e) reacting the reduced precursor catalyst with a tridentate ligand compound to obtain a catalyst and
f) optionally activating the catalyst issued from step e) with a cocatalyst.

2. A method according to claim 1 wherein the chromium content of the catalyst is 1 weight percent based on the total weight of the chromium and support.

3. A method according to claim 1 or 2 wherein the activation occurs at a temperature from 400 to 900°C.

4. A method according to any foregoing claims wherein the reduction occurs under carbon monoxide at a temperature of from 200°C to 600°C.

5. A method according to any foregoing claims wherein the tridentate ligand compound is a cyclic tridentate ligand with electron donating atoms in the cycle, substituted or unsubstituted.

6. A method according to claim 5 wherein the tridentate ligand compound is a neutral cyclic tridentate ligand with electron donating atoms in the cycle, substituted or unsubstituted.

7. A method according to any foregoing claims wherein the tridentate ligand compound is the 1,3,5-triazacyclohexane substituted or unsubstituted and the catalyst is further activated with a cocataly st.

8. A method according to claim 7 wherein the triazacyclohexane is 1,3,5-triazacyclohexane wherein each of the nitrogen atoms is independently substituted with hydrocarbon radicals.

9. A method according to claim 8 wherein the hydrocarbon radicals are benzyl

10. Heterogeneous chromium catalyst system obtainable according to claim 1 **characterised in that** the tridentate ligand is the 1,3,5-triazacyclohexane substitued or unsubstituted and that after treatment with said triazacyclohexane, the catalyst is further activated with a cocatalyst.

11. Heterogeneous chromium catalyst system according to claim 10 **characterised in that** the tridentate ligand is 1,3,5 triazacyclohexane wherein each of the nitrogen atoms is independently substituted with hydrocarbon radicals..

12. Heterogeneous chromium catalyst system according to claim 11 **characterised in that** hydrocarbon radicals are benzyl.

13. A process for the polymerisation of ethylene and/or alpha olefins comprising the steps of passing the ethylene and/or alpha olefins over the heterogeneous chromium catalyst system according to any one of claims 10 to 12 and further recovering the polymer.

14. A process according to claim 13 for the polymerisation of ethylene to hexene.
